(19) Europäisches Patentamt
European Patent Office
Office européen des brevets

(11) Veröffentlichungsnummer: 0 181 589
A2

(12) # EUROPÄISCHE PATENTANMELDUNG

(21) Anmeldenummer: 85114022.8

(51) Int.Cl.⁴: C 07 D 239/47

(22) Anmeldetag: 05.11.85

(30) Priorität: 14.11.84 DE 3441524

(43) Veröffentlichungstag der Anmeldung:
21.05.86 Patentblatt 86/21

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Baasner, Bernd, Dr.
Hamberger Strasse 27 d
D-5090 Leverkusen 3(DE)

(72) Erfinder: Klauke, Erich, Dr.
Eichendorffweg 8
D-5068 Odenthal(DE)

(54) **Verfahren zur Herstellung von 5-Fluorcytosin.**

(57) 5-Fluorcytosin wird hergestellt, indem man 2,5-Difluor-4-chlor-pyrimidin mit einen Protonensäure in Gegenwart von Wasser zu 2-Hydroxy-4-chlor-5-fluorpyrimidin und dieses mit Ammoniak zu 5-Fluorcytosin umsetzt.



Croydon Printing Company Ltd

BAYER AKTIENGESELLSCHAFT 5090 Leverkusen, Bayerwerk

Konzernverwaltung RP
Patentabteilung Gai/by-c

# Verfahren zur Herstellung von 5-Fluorcytosin

Die vorliegende Erfindung betrifft ein neues Verfahren zur Herstellung von 5-Fluorcytosin, das auch als 2-Hydroxy-4-amino-5-fluorpyrimidin bezeichnet werden kann.

5-Fluorcytosin ist als Pharmazeutikum und als Zwischenprodukt zur Herstellung von pharmakologisch wirksamen Substanzen bekannt (siehe z.B. R. Filler und S. M. Naqvi in "Biomedicinal Aspects of Fluorine Chemistry", Eds. R. Filler und Y. Kobayashi, Elsevier, Amserdam-New York-Oxford, 1982). Die Herstellung erfolgte bisher durch Direktfluorierung von Cytosin (siehe US-PS 3 846 429 und CA-PS 985 681), was einen technisch aufwendigen Umgang mit elementarem Fluor erfordert, oder durch aufwendige Ringschlußreaktionen von α-Fluor-ß-ketoesterenolaten mit den Salzen von Isothioharnstoffen (siehe J. Am. Chem. Soc. 79, 4559 (1957)).

Eine weitere Synthese geht von 5-Fluoruracil aus, einer Verbindung, die gleichfalls nur aufwendig durch Direktfluorierung von Uracil mit elementarem Fluor herzustellen ist (siehe US-PS 3 846 429 und CA-PS 985 681) oder durch aufwendige Ringschlußreaktionen (siehe J. Am. Chem. Soc. 79, 4559 (1957)). Das 5-Fluoruracil muß dann noch weiter mit Phosphoroxychlorid in das 2,4-Dichlor-5-fluor-pyrimidin überführt werden. Anschließend wird in der 4-Position die Aminogruppe, dann in der 2-Position die Hydroxygruppe eingeführt und somit abschließend das 2-Hydroxy-4-amino-5-fluorpyrimidin (≙ 5-Fluorcytosin) erhalten (siehe GB-PS 877 318). Dieser Weg umfaßt viele einzelne Stufen, ist daher sehr aufwendig und geht von dem schwer zugänglichen 5-Fluoruracil aus.

Weiterhin ist es bekannt, 2-Hydroxy-4-chlor-5-fluor-pyrimidin - diese Verbindung wird bei dem erfindungsgemäßen Verfahren als Zwischenstufe durchlaufen - zu synthetisieren, indem wiederum ausgehend vom 5-Fluor-uracil dieses mit $P_2S_5$ in der 4-Position geschwefelt und die danach erhaltene Verbindung 5-Fluorpyrimidin-2-on-4-thion anschließend mit Thionylchlorid in das 2-Hydroxy-4-chlor-5-fluorpyrimidin überführt wird (siehe Acta Chem. Scand. 23, 294 (1969)).

Dieses Verfahren hat den Nachteil, daß teurere Reagenzien als bei dem erfindungsgemäßen Verfahren eingesetzt werden und aufwendigere Aufarbeitungen und Entsorgungen vorgenommen werden müssen. Zudem beträgt die Ausbeute in der letzten Stufe lediglich 45 % der Theorie.

Es wurde nunmehr ein Verfahren zur Herstellung von 5-Fluorcytosin gefunden, das dadurch gekennzeichnet ist, daß man 2,5-Difluor-4-chlorpyrimidin mit einer Protonensäure in Gegenwart von Wasser zu 2-Hydroxy-4-chlor-5-fluorpyrimidin und dieses mit Ammoniak zu 2-Hydroxy-4-amino-5-fluorpyrimidin (≙ 5-Fluorcytosin) umsetzt.

Das erfindungsgemäße Verfahren kann durch folgende Reaktionsgleichung veranschaulicht werden:

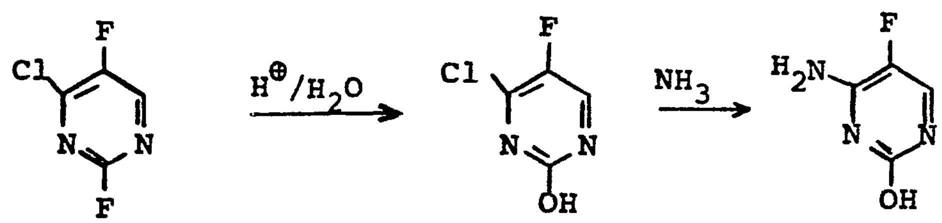


Erfindungsgemäß wird in einer ersten Stufe 2,5-Difluor-4-chlorpyrimidin mit einer Protonensäure in Gegenwart von Wasser umgesetzt. Die eingesetzte Protonensäure kann von beliebiger Art und Stärke sein. Bevorzugt werden starke Protonensäuren in Form von beliebig konzentrierten wäßrigen Lösungen eingesetzt. Insbesondere bevorzugt sind verdünnte und konzentrierte wäßrige Lösungen von Salzsäure, Schwefelsäure und Phosphorsäure.

Die Protonensäure und das Wasser sollten im allgemeinen in einer solchen Menge zugesetzt werden, daß pro Mol

2,5-Difluor-4-chlorpyrimidin mindestens ein Mol Protonen und mindestens ein Mol Wasser zur Verfügung stehen. Überschüsse, auch größere, an Protonensäure und/oder Wasser stören nicht. Aus wirtschaftlichen Gründen ist der Einsatz von 1 bis 2 Molen Protonensäure und 2 bis 100 Molen Wasser, jeweils pro Mol 2,5-Difluor-4-chlorpyrimidin, bevorzugt.

Geeignete Reaktionstemperaturen für diese Reaktionsstufe sind beispielsweise solche im Bereich von 0 bis 150°C. Bevorzugt sind Temperaturen im Bereich von 0 bis 100°C.

Auf diese Weise erhält man im allgemeinen in Ausbeuten von über 90 % der Theorie 2-Hydroxy-4-chlor-5-fluorpyrimidin. Dieses kann gegebenenfalls nach üblichen Methoden isoliert werden, beispielsweise indem man zunächst alle flüchtigen Bestandteile des Reaktionsgemisches bei Normaldruck oder im Vakuum entfernt, den Rückstand mit Wasser aufnimmt, dieses Gemisch neutral stellt und anschließend durch Einengen der Lösung das 2-Hydroxy-4-chlor-5-fluorpyrimidin ausfällt und abtrennt. Bei der Neutralstellung stellt man beispielsweise durch Zugabe einer wäßrigen Lösung von Ammoniak, eines Alkalicarbonats oder eines Alkalihydrogencarbonats einen pH-Wert von mindestens 7, vorzugsweise einen pH-Wert von 7 bis 8, ein.

Erfindungsgemäß wird in einer zweiten Stufe das 2-Hydroxy-4-chlor-5-fluorpyrimidin mit Ammoniak umgesetzt, wobei das

in 4-Position gebundene Chlor gegen eine Aminogruppe ausgetauscht wird. Die Ammoniakmenge kann in weiten Grenzen variiert werden. Beispielsweise können 1 bis 20 Mol Ammoniak pro Mol 2-Hydroxy-4-chlor-5-fluorpyrimidin eingesetzt werden. Bevorzugt ist der Einsatz von 1 bis 8 Mol Ammoniak pro Mol 2-Hydroxy-4-chlor-5-fluorpyrimidin.

Die Umsetzung mit Ammoniak kann auf die verschiedensten Arten vorgenommen werden. Beispielsweise kann man den Ammoniak in kondensierter Form einsetzen, indem man ihn bei Temperaturen unterhalb seines Siedepunktes mit 2-Hydroxy-4-chlor-5-fluorpyrimidin zusammenbringt. Vorzugsweise gibt man dabei 2-Hydroxy-4-chlor-5-fluorpyrimidin in kleinen Portionen, gegebenenfalls in gelöster Form, in kondensierten Ammoniak. Anschließend kann das Reaktionsgemisch erwärmt werden, z.B. auf Raumtemperatur oder höhere Temperaturen, wobei jedoch die Umsetzung in der Regel schon beim Erreichen einer Temperatur von +10°C beendet ist.

Der Ammoniak kann auch in Form von Lösungen beliebiger Konzentration eingesetzt werden, wobei Lösungen in organischen Lösungsmitteln wie Tetrahydrofuran, Diethylether oder Dioxan und Lösungen in Wasser bevorzugt sind. Dabei kann die Ammoniaklösung vorgelegt und das 2-Hydroxy-4-chlor-5-fluorpyrimidin, gegebenenfalls gelöst in einem Lösungsmittel wie Tetrahydrofuran, Diethylether, Dioxan, Methanol oder Ethanol, zugefügt oder umgekehrt verfahren werden.

Wenn man den Ammoniak in Form wäßriger Lösungen einsetzt sind konzentrierte Lösungen bevorzugt, beispielsweise solche, die 20 bis 33 Gew.-% Ammoniak enthalten. Weiterhin ist es in diesem Falle vorteilhaft, in Gegenwart eines mit Wasser mischbaren Lösungsmittels zu arbeiten, beispielsweise in Gegenwart von Methanol, Ethanol, Dioxan und/oder Tetrahydrofuran.

Die Temperatur für die Umsetzung mit Ammoniak kann beispielsweise im Bereich von -80 bis +60°C liegen. Bevorzugt sind Temperaturen im Bereich von -40 bis +30°C.

Besonders bevorzugt führt man die Umsetzung mit Ammoniak so durch, daß man eine konzentrierte wäßrige Lösung von Ammoniak zu einer Lösung von 2-Hydroxy-4-chlor-5-fluorpyrimidin in Methanol, Ethanol, Dioxan und/oder Tetrahydrofuran hinzufügt.

Die Umsetzung mit Ammoniak ist im allgemeinen nach 1 bis 8 Stunden beendet. Das dabei erhaltene 2-Hydroxy-4-amino-5-fluorpyrimidin (≙ 5-Fluorcytosin) fällt in den meisten Fällen während der Reaktion, gegebenenfalls auch erst nach Einengung des Reaktionsgemisches, in kristalliner Form aus dem Reaktionsgemisch aus und kann daraus in einfacher Weise durch Filtration und Trocknung gewonnen werden. Man kann auch so verfahren, daß man zunächst die flüchtigen Anteile aus dem nach der Umsetzung mit Ammoniak vorliegenden Gemisch im Vakuum durch Destillation entfernt, den Rückstand in wenig Wasser aufnimmt und das weitgehend ungelöst verbleibende

Produkt abfiltriert und trocknet. Die Reinheit des erhaltenen Produktes liegt in diesem Fall, ohne daß eine Umkristallisierung vorgenommen wird, meistens bei mehr als 98,5 %.

Zur Durchführung der zweiten Stufe des erfindungsgemäßen Verfahrens ist es nicht notwendig, nach der ersten Stufe das 2-Hydroxy-4-chlor-5-fluorpyrimidin zu isolieren. Man kann auch das gesamte nach der ersten Reaktionsstufe vorliegende Gemisch mit mindestens so viel Ammoniak versetzen, wie zur Neutralisation des Gemisches und für die Umsetzung des vorhandenen 2-Hydroxy-4-chlor-5-fluor-pyrimidins zu 2-Hydroxy-4-amino-5-fluorpyrimidin notwendig ist. Vorzugsweise wendet man dabei den Ammoniak in Form einer konzentrierten wäßrigen Lösung an und arbeitet in Gegenwart eines mit Wasser mischbaren Lösungsmittels, wie Methanol, Ethanol, Tetrahydrofuran und/oder Dioxan. Nach der oben beschriebenen Aufarbeitung erhält man dann ebenfalls meistens Produkte in Reinheiten von mehr als 98,5 %.

Unabhängig davon, ob das 2-Hydroxy-4-chlor-5-fluorpyrimidin nach der ersten Stufe isoliert wird oder nicht, erhält man gemäß dem zweistufigen erfindungsgemäßen Verfahren 2-Hydroxy-4-amino-5-fluorpyrimidin (≙ Fluorcytosin) im allgemeinen in Ausbeuten von 88 bis 93 % der Theorie (gerechnet über beide Stufen).

Das erfindungsgemäße Verfahren und die damit erzielbaren Vorteile sind ausgesprochen überraschend, da

gemäß der GB-PS 877 318 zu erwarten war, daß die erste nucleophile Umsetzung des 2,5-Difluor-4-chlorpyrimidins wenn nicht ausschließlich, dann jedoch zumindest ganz überwiegend in der 4-Position stattfindet, und somit das 2,5-Difluor-4-hydroxy-pyrimidin als Produkt der ersten Stufe zu erwarten war.

Das zur Durchführung des erfindungsgemäßen Verfahrens als Ausgangsprodukt benötigte 2,5-Difluor-4-chlor-pyrimidin ist gemäß einer eigenen älteren Patentanmeldung durch die partielle selektive Hydrierung des 2,5-Difluor-4,6-dichlorpyrimidins auf einfache Weise zugänglich (siehe das folgende Beispiel 1).

Die folgenden Beispiele erläutern das erfindungsgemäße Verfahren, ohne es in irgendeiner Weise zu beschränken.

## Beispiele

### Beispiel 1 (nicht erfindungsgemäß)

Herstellung von 2,5-Difluor-4-chlorpyrimidin aus 2,5-Difluor-4,6-dichlorpyrimidin

185 g (1 Mol) 2,5-Difluor-4,6-dichlorpyrimidin wurden in 1800 ml Essigsäureethylester unter Zusatz von 110 g Triethylamin und 15 g Palladium auf Kohle (5 gew.-%ig) bei 30°C und einem Wasserstoffdruck von 3,5 bar in einem Edelstahl-Rührautoklaven während 95 Minuten hydriert. Anschließend wurden die festen Anteile des Reaktionsgemisches abfiltriert, der Rückstand mit Essigsäureethylester gewaschen und das mit der Waschflüssigkeit vereinigte Filtrat bei Normaldruck über eine 30 cm-Füllkörperkolonne destilliert. Nachdem das Lösungsmittel abdestilliert war, wurden 106 g 2,5-Difluor-4-chlorpyrimidin mit einem Siedepunkt von 145 bis 146°C erhalten. Die Ausbeute betrug demnach 70,5 % der Theorie. Nach gaschromatographischer Analyse wies das isolierte Reaktionsprodukt eine Reinheit von 94,7 % auf.

Das für diese Herstellung von 2,5-Difluor-4-chlorpyrimidin benötigte 2,5-Difluor-4,6-dichlorpyrimidin ist ebenfalls gemäß einer eigenen älteren Patentanmeldung zugänglich, beispielsweise indem man das bekannte Tetrafluorpyrimidin mit Chlorwasserstoffgas bei erhöhtem Druck und erhöhter Temperatur (z.B. 30 bar Chlorwasserstoff und 160°C) umsetzt und das dabei erhaltene Gemisch fraktioniert destilliert.

Beispiel 2

Herstellung von 2-Hydroxy-4-chlor-5-fluorpyrimidin

15,05 g (0,1 Mol) 2,5-Difluor-4-chlorpyrimidin wurden zusammen mit 30 ml 37 %iger wäßriger Salzsäure 2 Stunden zum Rückfluß erhitzt. Anschließend wurden die flüchtigen Bestandteile im Vakuum abgezogen, der Rückstand in 100 ml Wasser aufgenommen und dieses Gemisch mit 20 %igem wäßrigem Ammoniak schwach alkalisch gestellt (pH 7,5). Es wurde dann erneut im Vakuum bis auf 20 % des ursprünglichen Volumens eingeengt. Der angefallene Feststoff wurde abgesaugt und getrocknet. Es wurden 13,7 g Produkt mit einem Schmelzpunkt von 176 bis 177°C (Zersetzung) erhalten, was einer Ausbeute von 92,2 % entspricht.

Beispiel 3

Herstellung von 5-Fluorcytosin aus 2-Hydroxy-4-chlor-5-fluorpyrimidin

14,85 g (0,1 Mol) 2-Hydroxy-4-chlor-5-fluorpyrimidin, das gemäß Beispiel 2 erhalten wurde, wurden in 100 ml Ethanol suspendiert. Dazu wurden unter Rühren bei Raumtemperatur 20 ml 33 %ige wäßrige Ammoniaklösung zugetropft und 90 Minuten nachgerührt. Anschließend wurde im Vakuum auf ein Drittel des ursprünglichen Volumens eingeengt, der angefallene Feststoff abgesaugt, mit Wasser nachgewaschen und getrocknet. Es wurden 12,64 g Produkt mit einem Schmelzpunkt von 294 bis 295°C erhalten, was einer Ausbeute von 98 % der Theorie entspricht.

Beispiel 4

Herstellung von 5-Fluorcytosin aus 2,5-Difluor-4-chlorpyrimidin

15,05 g (0,1 Mol) 2,5-Difluor-4-chlorpyrimidin wurden in 30 ml 37 %iger wäßriger Salzsäure 2 Stunden bei 50°C gerührt. Nach dem Erkalten wurde mit 33 %iger wäßriger Ammoniaklösung neutralisiert. Anschließend wurde mit 100 ml Ethanol verdünnt, dann weitere 20 ml wäßrige, 33 %ige Ammoniaklösung zugesetzt. Man rührte bei Raumtemperatur 2 Stunden nach. Danach wurde im Vakuum eingeengt, der Rückstand in 60 ml Wasser aufgenommen, der angefallene Feststoff abgesaugt, mit Wasser gewaschen und getrocknet. Es wurden 11,5 g Produkt mit einem Schmelzpunkt von 294 bis 296°C erhalten, was einer Ausbeute von 98,1 % der Theorie entspricht.

Patentansprüche

1. Verfahren zur Herstellung von 5-Fluorcytosin, dadurch gekennzeichnet, daß man 2,5-Difluor-4-chlorpyrimidin mit einer Protonensäure in Gegenwart von Wasser zu 2-Hydroxy-4-chlor-5-fluorpyrimidin und dieses mit Ammoniak zu 2-Hydroxy-4-amino-5-fluorpyrimidin (≙ 5-Fluorcytosin) umsetzt.

2. Verfahren nach Anspruch 1, dadurch gekennzeichnet, daß man eine starke Protonensäure einsetzt.

3. Verfahren nach Ansprüchen 1 und 2, dadurch gekennzeichnet, daß man eine wäßrige Lösung von Salzsäure, Schwefelsäure oder Phosphorsäure einsetzt.

4. Verfahren nach Ansprüchen 1 bis 3, dadurch gekennzeichnet, daß man pro Mol 2,5-Difluor-4-chlorpyrimidin 1 bis 2 Mole einer Protonensäure und 2 bis 100 Mole Wasser einsetzt.

5. Verfahren nach Ansprüchen 1 bis 4, dadurch gekennzeichnet, daß man die Umsetzung mit der Protonensäure bei Temperaturen im Bereich von 0 bis 150°C durchführt.

6. Verfahren nach Ansprüchen 1 bis 5, dadurch gekennzeichnet, daß man pro Mol 2-Hydroxy-4-chlor-5-fluorpyrimidin 1 bis 20 Mole Ammoniak einsetzt.

7. Verfahren nach Ansprüchen 1 bis 6, dadurch gekennzeichnet, daß man die Umsetzung mit Ammoniak bei Temperaturen im Bereich -80 bis +60°C durchführt.

8. Verfahren nach Ansprüchen 1 bis 7, dadurch gekennzeichnet, daß man den Ammoniak in Form einer 20 bis 33 gew.-%igen wäßrigen Lösung einsetzt und in Gegenwart eines mit Wasser mischbaren Lösungsmittels arbeitet.

9. Verfahren nach Ansprüchen 1 bis 8, dadurch gekennzeichnet, daß man das 2-Hydroxy-4-chlor-5-fluorpyrimidin nicht isoliert, sondern das gesamte, nach der Umsetzung mit einer Protonensäure vorliegende Gemisch mit Ammoniak versetzt.

10. Verfahren nach Anspruch 9, dadurch gekennzeichnet, daß man 20 bis 33 gew.-%igen wäßrigen Ammoniak einsetzt und in Gegenwart eines mit Wasser mischbaren Lösungsmittels arbeitet.